(19) Europäisches Patentamt
European Patent Office
Office européen des brevets

(11) Veröffentlichungsnummer: **0 207 445**
**B1**

(12) **EUROPÄISCHE PATENTSCHRIFT**

(45) Veröffentlichungstag der Patentschrift: **12.10.88**

(51) Int. Cl.⁴: **C 07 C 143/44**, C 07 C 143/46, C 07 C 139/00

(21) Anmeldenummer: **86108695.7**

(22) Anmeldetag: **26.06.86**

(54) **Verfahren zur Herstellung von Acyloxybenolsulfonsäuren und deren Salzen.**

(30) Priorität: **05.07.85 DE 3524052**

(43) Veröffentlichungstag der Anmeldung: **07.01.87 Patentblatt 87/2**

(45) Bekanntmachung des Hinweises auf die Patenterteilung: **12.10.88 Patentblatt 88/41**

(84) Benannte Vertragsstaaten: **AT BE CH DE FR GB IT LI NL SE**

(56) Entgegenhaltungen:
**EP-A-0 140 251**
**US-A-3 503 888**

(73) Patentinhaber: **HOECHST AKTIENGESELLSCHAFT, Postfach 80 03 20, D-6230 Frankfurt am Main 80 (DE)**

(72) Erfinder: **Grabley, Fritz- Feo, Dr., Hölderlinstrasse 7, D-6240 Königstein/Taunus (DE)**
Erfinder: **Reinardt, Gerd, Dr., Freiherr- vom- Stein- Strasse 37, D-6233 Kelkheim (Taunus) (DE)**
Erfinder: **Steinl, Roland, Langstrasse 24, D-6232 Bad Soden am Taunus (DE)**

**EP 0 207 445 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von Acyloxybenzolsulfonsäuren und deren Salzen in einer mehrstufigen Reaktionsfolge ausgehend von Phenol.

Acyloxybenzolsulfonsäuren und ihre Salze sind seit langem bekannte Verbindungen mit tensidischer Wirkung, für die es mehrere Möglichkeiten zur Herstellung gibt.

Bereits in der DE-C-666 626 ist ein Verfahren beschrieben, bei dem Ester aus Phenolen und gesättigten Alkanmonosäuren mit mindestens 10 C-Atomen mit einem Sulfierungsmittel wie Chlorsulfonsäure, Oleum oder $SO_3$ umgesetzt, und das Reaktionsprodukt anschließend neutralisiert wird. Während, vor oder nach der Sulfierung wird eine kleine Menge an Phosphorchlorid, -oxychlorid oder Thionylchlorid zugesetzt. Die Reaktionsprodukte sollen beispielsweise als Waschmittel Verwendung finden können.

Aus der US-A-3 320 292 ist ein Verfahren bekannt, bei dem Hydroxyalkansulfonate oder Hydroxybenzolsulfonate direkt mit organischen ($C_8$-$C_{20}$)Alkansäuren bei 200 bis 240° C in Gegenwart von Zinkoxid oder organischen Zinksalzen verestert werden; in den Beispielen werden allerdings keine aromatischen Sulfonate eingesetzt. Die Reaktionsprodukte sollen tensidische Wirkung zeigen, beispielsweise als Seifenkomponente. Gemäß der US-A-3 394 155 wird diese Veresterung in zwei Stufen in Gegenwart von Veresterungshilfsmitteln wie Zinnsulfat, Chloressigsäure oder den vorstehend genannten Zinkverbindungen durchgeführt.

Nach den Angaben in der US-A-3 503 888 wird das Verfahren so durchgeführt, daß a) Phenol zuerst mit $SO_3$ in einem inerten organischen Lösemittel sulfiert, b) das sulfierte Phenol mit einem Fettsäurechlorid verestert, und c) der Ester dann mit NaOH auf einen pH-Wert von 6,5 bis 9 neutralisiert wird; bei diesem Verfahren ist während der Neutralisation als Nebenreaktion eine teilweise Hydrolyse des gebildeten Esters im Umfang von 20 bis 50 % beabsichtigt. Die Verfahrensprodukte werden als Seifenkomponente verwendet. Dagegen wird dieses Verfahren in der EP-A-0 140 251 ohne Esterverseifung beschrieben, wonach die Neutralisationsstufe (Salzbildungsstufe des Esters) mit Alkali- oder Erdalkalihydroxiden, -carbonaten oder -bi-carbonaten bei einem pH-Wert von 2,5 bis 7,0 durchgeführt wird.

In jüngster Zeit haben Acyloxybenzolsulfonate auch Interesse als Perborataktivatoren in Waschmitteln gefunden, siehe dazu beispielsweise die EP-A-0 098 129, EP-A-0 105 672, EP-A-0 105 673 und EP-A-0 125 641, wobei die Acyloxybenzolsulfonate im wesentlichen nach folgenden Methoden synthetisiert werden:

- Erhitzen eines Gemisches aus Trifluoressigsäureanhydrid, Na-phenolsulfonat und einer ($C_6$-$C_{19}$)Alkansäure,
- zweistufige Umsetzung durch a) Umwandlung der Alkansäure in das Anhydrid in einem Überschuß an Essigsäureanhydrid, und b) Reaktion des isolierten Anhydrids mit Na-phenolsulfonat,
- Erhitzen des Alkansäurechlorids (z. B. Nonanoylchlorid) mit Na-phenolsulfonat bei 80 bis 100° C in einem organischen aprotischen Lösemittel wie Dioxan, Dichlorethan oder Toluol unter Anwendung eines $N_2$-Stroms zur Entfernung des Nebenproduktes HCl,
- Umesterung eines ($C_2$-$C_3$)Acyloxybenzolsulfonats mit einer ($C_6$-$C_{18}$)Alkansäure im Überschuß und Entfernung des entstehenden Nebenprodukts ($C_2$-$C_3$)Alkansäure,
- mehrstufige "Eintopf"-Umsetzung durch a) Umwandlung einer ($C_6$-$C_{18}$)Alkansäure in das Anhydrid in einem ($C_2$-$C_3$)Alkansäureanhydrid, b) Entfernung der gebildeten ($C_2$-$C_3$)Alkansäure, c) Reaktion des Anhydrids mit einem Phenolsulfonat in Gegenwart einer starken Säure oder Base, und d) Isolierung des ($C_6$-$C_{18}$)Acyloxybenzolsulfonats aus dem Reaktionsgemisch, oder
- Umsetzung eines Alkalimetall- oder Erdalkalimetallphenolsulfonats mit einem ($C_2$-$C_{31}$)Alkansäurephenylester bei 200 bis 350° C.

Die bekannten Methoden haben jedoch - wie auch die Vielzahl von Verfahrensvarianten zeigt - mehr oder weniger große Nachteile, die teilweise auch noch vom vorgesehenen Verwendungszweck der Verfahrensprodukte abhängen: Oftmals laufen die Reaktionsstufen bei Temperaturen von mehr als 200° C ab, so daß die Gefahr von Nebenreaktionen und Zersetzungen (Verfärbungen) besteht. Bei der Sulfierung treten häufig Isomerengemische auf, die dann bei einer nachfolgenden Reaktion oder bei einer direkten Verwendung der Sulfierungsprodukte weniger wirksam sind. Einige der Varianten (beispielsweise Umesterungen) sind zwar im Labormaßstab durchführbar, führen aber bei ihrer Übertragung in den Produktionsmaßstab zu ökonomischen und/oder auch ökologischen Problemen.

Aufgabe der vorliegenden Erfindung ist es deshalb, ein auch im großtechnischen Maßstab durchführbares Verfahren aufzuzeigen, daß in guten Ausbeuten zu möglichst einheitlichen Produkten führt, die insbesondere auf dem Anwendungsgebiet eines Perborataktivators in Waschmitteln eingesetzt werden können.

Die Erfindung geht aus von dem bekannten Verfahren zur Herstellung von Acyloxybenzolsulfonsäuren der allgemeinen Formel

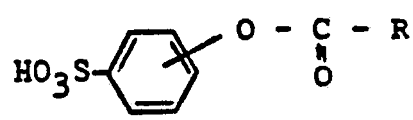

mit R in der Bedeutung eines ($C_1$-$C_{17}$)Alkyl- oder ($C_2$-$C_{17}$)Alkenylrestes, oder von deren

Alkalimetall-, Erdalkalimetall- oder Ammoniumsalzen mit den Stufen (a) Sulfierung von Phenol, (c) Veresterung des sulfierten Phenols und gegebenenfalls (d) Neutralisierung des Esters. Das erfindungsgemäße Verfahren ist dann dadurch gekennzeichnet, daß das Sulfierungszwischenprodukt der Stufe (a) vor der Veresterung in einer Stufe (b) bei einer Temperatur von 50 bis 110° C thermisch behandelt wird.

Die einzelnen Stufen des Verfahrens werden insbesondere - wie nachfolgend beschrieben - ausgeführt: In der Sulfierungsstufe (a) wird Phenol zur Phenolsulfonsäure umgesetzt, wobei im wesentlichen die in 4-Stellung substituierten neben den in 2-Stellung substituierten Verbindungen entstehen; daneben können aber noch - in untergeordneter Menge - Nebenprodukte wie Disulfonsäuren oder Sulfone entstehen. Als Sulfierungsmittel kommen bevorzugt $ClSO_3H$ oder $SO_3$ (flüssig oder gasförmig) in Betracht. Die Reaktion kann in Gegenwart von aprotischen organischen Lösemitteln wie halogenierten Kohlenwasserstoffen (z. B. Methylenchlorid), Toluol oder Essigsäureethylester durchgeführt werden; sie führt aber insbesondere auch in lösemittelfreien Systemen zum Erfolg, wobei dann beispielsweise in beiden Varianten auch organische Säuren beispielsweise ($C_2$-$C_{18}$)Alkansäuren wie $CH_3COOH$ oder Isononansäure und/oder anorganische Salze wie $Na_2SO_4$ oder $Na_2H_2P_2O_7$ zugesetzt werden können. Die Reaktionstemperatur liegt im allgemeinen bei 0° C bis 120° C, bevorzugt im Bereich von 30 bis 60° C. Die Reaktionszeiten betragen zweckmäßig 0,5 bis 8 h; die eingesetzte Menge an Sulfierungsmittel beträgt bevorzugt 0,8 bis 1,2, insbesondere 0,9 bis 1,1 Mol pro Mol Phenol.

In der erfindungsgemäß besonders wichtigen Temperungsstufe (b) wird das Sulfierungsprodukt der Stufe (a) thermisch so behandelt, daß der Anteil des in 4-Stellung sulfierten relativ zum Anteil des in 2-Stellung sulfierten Phenols und damit auch in absoluter Menge zunimmt. Dazu wird zunächst bevorzugt - sofern überhaupt in der Stufe (a) eingesetzt - das organische Lösemittel aus dem Reaktionsgemisch entfernt, und dann wird die gebildete Phenolsulfonsäure auf eine Temperatur von 50 bis 110° C erhitzt. Diese Stufe kann aber auch in Gegenwart von geeigneten aprotischen organischen Lösemitteln und/oder organischen Säuren und/oder anorganischen Salzen durchgeführt werden. Geeignete Beispiele für die genannten Zusätze sind bereits bei der Beschreibung der Stufe (a) aufgeführt, wobei angenommen wird, daß insbesondere der Zusatz von Alkansäuren und anorganischen Salzen die Möglichkeit der Bildung von Sulfonen aus den Sulfonsäuren verhindert. Der Anteil des 4-Isomeren steigt bei dieser Temperung von (bezogen auf den Mengenanteil des 2-Isomeren) etwa 2 bis 3 zu 1 ohne Temperungsstufe auf bevorzugt etwa 8 bis 20 zu 1 (je nach Temperungsbedingungen). Die Temperungszeit beträgt im allgemeinen 0,5 bis 10 h.

In der sich anschließenden Acylierungsstufe (c) wird die freie Phenolsulfonsäure verestert, wobei die Phenolsulfonsäuren der Stufen (a) und (b) mit ($C_2$-$C_{18}$)Alkansäurehalogeniden (insbesondere -chloriden) oder -anhydriden ohne Zusätze oder mit den freien Säuren oder ihren Anhydriden in Gegenwart wasserentziehender Mittel wie $SOCl_2$ oder $POCl_3$ umgesetzt werden. Diese Stufe kann in Anwesenheit von aprotischen organischen Lösemitteln durchgeführt werden, aber insbesondere auch eine lösemittelfreie Variante führt zum Erfolg. Die Reaktionstemperatur liegt im allgemeinen bei 0° bis 110° C, vorzugsweise bei 20 bis 80° C, die Reaktionszeit bei 0,5 bis 8 h. Die eingesetzte Menge an Acylierungsmittel beträgt bevorzugt 0,8 bis 1,2, insbesondere 0,9 bis 1,1 Mol pro Mol Sulfonsäure; wenn Anhydride zum Einsatz kommen reduziert sich diese Menge auf 0,4 bis 0,6 Mol. Das häufig eingesetzte wasserentziehende Mittel wird in der Regel im Überschuß zugegeben. Da bei der Veresterung gelegentlich mit dem Auftreten von Schaum gerechnet werden muß, empfiehlt sich in diesen Fällen der Zusatz eines Entschäumers, beispielsweise auf Silikonbasis. Der Veresterungsstufe kann sich eine Nachbehandlung mit $SOCl_2$ oder $POCl_3$ anschließen. Als geeignete Acylierungsmittel werden die sich von ($C_6$-$C_{18}$)Alkansäure ableitenden Verbindungen bevorzugt, z. B. von Isononansäure (3,5,5-Trimethylhexansäure), Nonansäure, 2-Ethyl-hexansäure, Dodecansäure, Hexadecansäure und Octadecansäure. Die Kohlenwasserstoffreste dieser Verbindungen können gesättigt oder ungesättigt und geradkettig oder verzweigt sein; die Carboxylgruppe bzw. eine sich davon ableitende funktionelle Gruppe kann auch als Substituent innerhalb des Kohlenwasserstoffrestes stehen, bevorzugt ist sie jedoch endständig. In der Praxis sind die Alkansäuren häufig Gemische von Verbindungen unterschiedlicher Kettenlänge oder sie enthalten mehr oder weniger große Anteile an ungesättigten Verbindungen, im Mittel sollen sie aber in den vorstehenden Bereich fallen.

Der Acylierungsstufe (c) schließt sich gegebenenfalls - sofern die Herstellung der Salze beabsichtigt ist - noch eine Neutralisationsstufe (d) an, bei der die Sulfonsäuregruppe in die Salzform überführt wird. Die Neutralisation - vorzugsweise in wäßrigem Medium - wird bei kontrollierten pH-Werten durchgeführt, um eine Hydrolyse der in Stufe (c) gebildeten Acyloxybenzolsulfonsäuren möglichst weitgehend zu verhindern; sie kann im Bereich von pH 2 bis 7,5 gefahren werden, bevorzugt wird jedoch ein Bereich von 3 bis 6. Die Temperatur beträgt in dieser Stufe zweckmäßig 0 bis 50° C und als Basen werden beispielsweise Alkalimetall-, Erdalkalimetall- oder Ammoniumhydroxide, -carbonate oder

-hydrogencarbonate eingesetzt.

Das nach Beendigung der Stufen (c) oder (d) erhaltene Produkt wird abschließend getrocknet, beispielsweise durch Sprühtrocknung. Das erfindungsgemäße Verfahren kann diskontinuierlich, aber auch kontinuierlich in auf diesem Arbeitsgebiet üblichen Apparaten durchgeführt werden; es weist insbesondere folgende Vorteile gegenüber dem Stand der Technik auf:

- Die Reaktionsstufen werden so durchgeführt und können so aufeinander abgestimmt werden, daß infolge der verhältnismäßig niedrigen Temperaturen möglichst wenig Nebenprodukte (Verunreinigungen, Verfärbungen) entstehen.
- Durch die eingeschobene Temperungsstufe wird das Verhältnis von 4- zu 2-Isomeren der als Zwischenprodukt entstehenden Phenolsulfonsäuren signifikant zum 4-Isomeren hin verschoben, wodurch die Wirksamkeit der Endprodukte, der Acyloxybenzolsulfonsäuren oder insbesondere der -sulfonate, als Perborataktivator in Waschmitteln gesteigert wird.
- Das erfindungsgemäße Verfahren ist im Labor-, Technikums- und großtechnischen Maßstab ökonomisch und ökologisch günstig durchführbar, da es in guten Ausbeuten zu hochwirksamen Produkten führt, bei deren Herstellung verhältnismäßig wenig störende Nebenprodukte anfallen.

In den folgenden Beispielen verhalten sich Gew.-Teile zu Vol.-Teilen wie kg zu $dm^3$, %-Angaben beziehen sich - wenn nichts anderes angegeben ist - immer auf das Gewicht. Unter dem Begriff WS-Gehalt ist in den folgenden Beispielen der Anteil an waschaktiver Substanz im Produkt zu verstehen, der durch 2-Phasen-Titration nach EPTON bestimmt wird. Alle Produkte weisen eine in der Praxis geforderte "Farbzahl" auf, d.h. sie sind wenig durch Verfärbungen (Nebenprodukte) verunreinigt.

**Vergleichsbeispiel V**

Das Beispiel beschreibt die Herstellung und Umsetzung von Phenolsulfonsäure ohne zwischengeschaltete Temperungsstufe (b): Es werden 94 Gew.-Teile Phenol bei einer Temperatur von 40 bis 45°C geschmolzen. Unter Kühlung werden 116,5 Gew.-Teile Chlorsulfonsäure ($ClSO_3H$) so zugetropft, daß die Reaktionstemperatur 50°C nicht übersteigt; danach wird während 1 h hei dieser Temperatur gerührt. Bei einer Temperatur von 45 bis 50°C werden 176,7 Gew.-Teile Isononansäurechlorid innerhalb von 3h zu der gebildeten Phenolsulfonsäure zugetropft, und das Reaktionsgemisch wird während 1h nachgerührt; während und nach Beendigung dieser Stufe (c) wird darauf geachtet, daß das entstehende HCl ausgetragen wird. Das praktisch in quantitativer Ausbeute erhaltene Reaktionsprodukt weist einen WS-Gehalt von 81 % auf. Nach im pH-Wert kontrollierter Neutralisation (pH-Bereich von 2,5 bis 6) mit wäßriger NaOH-Lösung bei etwa 20°C und Sprühtrocknung des Na-Salzes der Isononanoyloxybenzolsulfonsäure betragen der WS-Gehalt 84 % und das Verhältnis des 4- zum 2-Isomeren 2,8 zu 1.

**Vergleichsbeispiel V 2**

Es wird ebenfalls ohne Temperungsstufe (b) gefahren: Es werden 94 Gew.-Teile Phenol in 500 Vol.-Teilen Methylenchlorid gelöst, und bei einer Temperatur von 25°C werden 116,5 Gew.-Teile $ClSO_3H$ innerhalb von 1,5 h so zugetropft, daß eine Reaktionstemperatur von 30°C nicht überschritten wird. Das Gemisch wird während 1 h nachgerührt und die Veresterung erfolgt bei einer Temperatur von 30°C während 2,5 h durch Zugabe von 176,7 Gew.-Teilen Isononansäurechlorid. Nach Entfernung des organischen Lösemittels durch Destillation wird praktisch in quantitativer Ausbeute die Isononanoyloxybenzolsulfonsäure isoliert, die nach weiterer Aufarbeitung gemäß V 1 als Na-Salz vorliegt und einen WS-Gehalt von 84 % und ein Isomerenverhältnis von 3,5 : 1 zeigt.

**Beispiel 1**

Es wird nach den Angaben des Vergleichsbeispiels V 1 verfahren, aber vor der Veresterung wird eine Temperungsstufe zwischengeschaltet, wobei die rohe Phenolsulfonsäure während 2 h bei 80 bis 83°C thermisch behandelt wird. Das Na-Salz als Endprodukt weist einen WS-Gehalt von 88 % und ein Isomerenverhältnis von 9 : 1 auf.

**Beispiel 2**

Es wird nach den Angaben des Beispiels 1 verfahren, aber die Temperungsstufe bei 95°C während 1,5 h durchgeführt. Das Na-Salz weist einen WS-Gehalt von 84,5 % und ein Isomerenverhältnis von 15,5 : 1 auf.

**Beispiel 3**

Es wird nach den Angaben des Beispiels 1 ohne Neutralisationsstufe (d) verfahren, aber in der Stufe (c) werden 224 Gew.-Teile Dodecansäurechlorid zugetropft. Die als Endprodukt dieses Beispiels erhaltene Dodecanoyloxybenzolsulfonsäure weist einen

WS-Gehalt von 85,3 % und ein Isomerenverhältnis von 8,5 : 1 auf.

**Beispiel 4**

Es wird nach den Angaben des Beispiels 1 verfahren, aber die Temperungsstufe (b) wird während 2,5 h durchgeführt, außerdem die Veresterungsstufe (c) mit 166 Gew.-Teilen Isononansäure und einem geringen Zusatz an einem Entschäumer auf Silikonbasis und 130 Gew.-Teilen an zugetropftem Thionylchlorid ($SOCl_2$) bei einer Temperatur von 35° C. Das Na-Salz weist einen WS-Gehalt von 89 % und ein Isomerenverhältnis von 9,5 zu 1 auf.

**Vergleichsbeispiel V 3**

Es werden zu 94 Gew.-Teilen Phenol bei einer Temperatur von 40° C in 158,5 Gew.-Teilen Isononansäure 122 Gew.-Teile $ClSO_3H$ zugegeben und es wird bei 40° C während 1 h nachgerührt. Danach werden 125 Gew.-Teile $SOCl_2$ so zudosiert, daß die Gasentwicklung kontrolliert abläuft, wonach noch während 1 h nachgerührt wird. Das nach weiterer Aufarbeitung gemäß V 1 erhaltene Na-Salz der Isononanoyloxybenzolsulfonsäure hat einen WS-Gehalt von 85 % und ein Isomerenverhältnis von 3,3 zu 1.

**Beispiele 5 bis 7**

Es wird nach den Angaben des Vergleichsbeispiels V 3 verfahren, aber vor der Veresterungsstufe (c) eine Temperungsstufe (b) zwischengeschaltet: 2 h bei einer Temperatur von 80° C (Bsp. 5), 4 h bei 80° C (Bsp. 6) und 1 h bei 95° C (Bsp. 7). Die WS-Gehalte bzw. Isomerenverhältnisse der Produkte sind: 87 % und 5,2 zu 1, 86 % und 7 zu 1, 86 % und 8,3 zu 1.

**Beispiel 8**

Die Phenolsulfonsäure wird nach den Angaben des Vergleichsbeispiels V 1 hergestellt und während 3 h bei einer Temperatur von 83° C behandelt. Das Zwischenprodukt wird in 146,8 Gew.-Teilen 2-Ethylhexansäure gelöst und anschließend durch Zugabe von 128,5 Gew.-Teilen Thionylchlorid bei 40 bis 45° C verestert, wonach während 1 h nachgerührt wird. Das nach weiterer Aufarbeitung gemäß V 1 erhaltene Na-Salz der 2-Ethyl-hexanoyloxybenzolsulfonsäure weist einen WS-Gehalt von 86,8 % und ein Isomerenverhältnis von 12 zu auf.

**Beispiel 9**

Es werden 94 Gew.-Teile Phenol in 158 Gew.-Teilen Isononansäure gelöst und dazu 80 Gew.-Teile flüssiges $SO_3$ bei einer Temperatur von 5° C zugetropft. Das Gemisch wird in der Stufe (b) während 6h bei einer Temperatur von 80° C behandelt. Die Veresterung erfolgt durch Zugabe von 125 Gew.-Teilen $SOCl_2$ bei 40 bis 45° C, wobei noch während 1 h nachgerührt wird. Das nach weiterer Aufarbeitung gemäß V 1 erhaltene Na-Salz der Isononanoyloxybenzolsulfonsäure weist einen WS-Gehalt von 85 % und ein Isomerenverhältnis von 8,7 : 1 auf.

**Patentansprüche**

1. Verfahren zur Herstellung von Acyloxybenzolsulfonsäuren der allgemeinen Formel

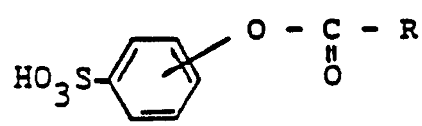

mit R in der Bedeutung eines ($C_1$-$C_{17}$)Alkyl- oder ($C_2$-$C_{17}$)Alkenylrestes, oder von deren Alkalimetall-, Erdalkalimetall- oder Ammoniumsalzen mit den Stufen (a) Sulfierung von Phenol, (c) Veresterung des sulfierten Phenols und gegebenenfalls (d) Neutralisierung des Esters, dadurch gekennzeichnet, daß das Sulfierungszwischenprodukt der Stufe (a) vor der Veresterung in einer Stufe (b) bei einer Temperatur von 50 bis 110° C thermisch behandelt wird.

2. Verfahren nach Anspruch 1, dadurch gekennzeichnet, daß die thermische Behandlung in der Stufe (b) bei einer Temperatur von 50 bis 110° C, gegebenenfalls in Gegenwart von aprotischen organischen Lösemitteln und/oder von organischen Säuren und/oder von anorganischen Salzen durchgeführt wird.

3. Verfahren nach Anspruch 1 oder 2, dadurch gekennzeichnet, daß sich der Acyloxyrest in den Acyloxybenzolsulfonsäure-Molekülen in der Stellung 2 oder 4 zum Sulfonsäurerest befindet.

4. Verfahren nach einem der Ansprüche 1 bis 3, dadurch gekennzeichnet, daß die Sulfierung in der Stufe (a) mit $ClSO_3H$ oder $SO_3$, gegebenenfalls in Gegenwart von aprotischen organischen Lösemitteln und/oder von organischen Säuren und/oder von anorganischen Salzen bei einer Temperatur von 30 bis 60° C durchgeführt wird.

5. Verfahren nach einem der Ansprüche 1 bis 4,

dadurch gekennzeichnet, daß die Veresterung in der Stufe (c) mit ($C_2$-$C_{18}$)Alkansäuren oder deren reaktionsfähigen Derivaten aus der Gruppe der Säurehalogenide und Säureanhydride, gegebenenfalls in Gegenwart von wasserentziehenden Mitteln und/oder von aprotischen organischen Lösemitteln bei einer Temperatur von 20 bis 80° C durchgeführt wird.

6. Verfahren nach einem der Ansprüche 1 bis 5, dadurch gekennzeichnet, daß die Neutralisierung in der Stufe (d) unter Kontrollierung des pH-Wertes in einem pH-Bereich von 3 bis 6 und einer Temperatur von 0 bis 50° C mit einer Base aus der Gruppe Alkalimetall-, Erdalkalimetall- und Ammoniumhydroxide, -carbonate und -hydrogencarbonate durchgeführt wird.

## Claims

1. A process for the preparation of an acyloxybenzenesulfonic acid of the formula

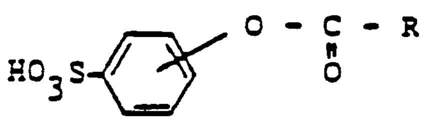

in which R denotes a ($C_1$-C17)-alkyl or ($C_2$-$C_{17}$)-alkenyl radical, or alkali metal, alkaline earth metal or ammonium salts thereof, employing the stages (a) sulfonation of phenol, (c) esterification of the sulfonated phenol and, if appropriate, (d) neutralization of the ester, which comprises subjecting the sulfonation intermediate product of stage (a) to heat treatment at a temperature of 50 to 110° C in a stage (b) before the esterification.

2. The process as claimed in claim 1, wherein the heat treatment in stage (b) is carried out at a temperature of 50 to 110° C, if appropriate in the presence of aprotic organic solvents and/or organic acids and/or inorganic salts.

3. The process as claimed in claim 1 or 2, wherein the acyloxy radical in the acyloxybenzenesulfonic molecules is located in position 2 or 4 relative to the sulfonic acid radical.

4. The process as claimed in any of claims 1 to 3, wherein the sulfonation in stage (a) is carried out at a temperature of 30 to 60° C by means of $ClSO_3H$ or $SO_3$, if appropriate in the presence of aprotic organic solvents and/or organic acids and/or inorganic salts.

5. The process as claimed in any of claims 1 to 4, wherein the esterification in stage (c) is carried out at a temperature of 20 to 80° C by means of ($C_2$-$C_{18}$)-alkanoic acids or reactive derivatives thereof selected from the group consisting of acid halides and acid anhydrides, if appropriate in the presence of dehydrating agents and/or aprotic organic solvents.

6. The process as claimed in claim 1, wherein the neutralization in stage (d) is carried out by means of a base selected from the group consisting of alkali metal hydroxides, carbonates and bicarbonates, alkaline earth metal hydroxides, carbonates and bicarbonates and ammonium hydroxides, carbonates and bicarbonates, with the pH controlled within a range from 3 to 6 and at a temperature of 0 to 50° C.

## Revendications

1. Procédé de préparation d'acides acyloxybenzène-sulfoniques de formule générale:

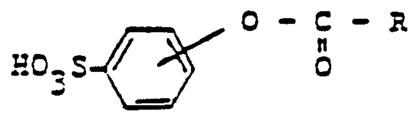

R désignant un alkyle en $C_1$-$C_{17}$ ou un alcényle en $C_2$-$C_{17}$, ou de leurs sels de métaux alcalins ou alcalino-terreux ou d'ammonium, par (a) sulfonation de phénol, (c) estérification du phénol sulfoné et le cas échéant (d) neutralisation de l'ester formé, procédé caractérisé en ce que l'on traite par la chaleur entre 50 et 110° C dans une étape (b), avant l'estérification, le produit intermédiaire de la sulfonation provenant de l'étape (a).

2. Procédé selon la revendication 1 caractérisé en ce que l'on effectue le traitement thermique de l'étape (b) entre 50 et 110° C en présence de solvants organiques aprotiques et/ou d'acides organiques et/ou de sels minéraux.

3. Procédé selon la revendication 1 ou 2 caractérisé en ce que le radical acyloxy de l'acide acyloxybenzène-sulfonique se trouve à la position 2 ou 4 par rapport au groupe sulfonique.

4. Procédé selon l'une quelconque des revendications 1 à 3, caractérisé en ce que l'on effectue la sulfonation de l'étape (a) avec $ClCO_3H$ ou $SO_3$, éventuellement en présence de solvants organiques aprotiques et/ou d'acides organiques et/ou de sels minéraux, à une température de 30 à 60° C.

5. Procédé selon l'une quelconque des revendications 1 à 4, caractérisé en ce que l'on effectue l'estérification de l'étape (c) avec des alcanoïques en $C_2$-$C_{18}$ ou des dérivés réactifs de ces acides qui sont des halogénures ou les anhydrides, éventuellement en présence d'agents déshydratants et/ou de solvants organiques aprotiques, à des températures de 20 à 80° C.

6. Procédé selon l'une quelconque des revendications 1 à 5, caractérisé en ce que l'on effectue la neutralisation (d) à un pH réglé entre 3 et 6 et à une température de 0 à 50° C, avec une base prise parmi les hydroxydes, carbonates et hydrogénocarbonates de métaux alcalins ou alcalino-terreux ou d'ammonium.